# EUROPEAN PATENT SPECIFICATION

(11) **EP 2 346 559 B1**
(45) Date of publication and mention of the grant of the patent: **28.08.2013**
(21) Application number: 09756344.9
(22) Date of filing: 09.10.2009
(51) Int. Cl.: A61M 25/00

(54) **MEDICAL DEVICE**
MEDIZINISCHE VORRICHTUNG
DISPOSITIF MÉDICAL

(30) Priority: 09.10.2008 US 104127 P
(43) Date of publication of application: 27.07.2011
(73) Proprietor: NYXX TECHNOLOGIES LIMITED, Stratford upon Avon Warwickshire CV37 6JG (GB)
(72) Inventor: WYLLIE, Michael Grant, Faversham, Kent ME13 7BN (GB); TIHON, Claude, Minnesota 55347 (US)
(74) Representative: Parnham, Kevin
(86) International application number: PCT/GB2009/051353
(87) International publication number: WO 2010/041084

(56) References cited:
- WO-A1-93/10845
- WO-A1-96/08287
- WO-A1-2004/056414
- WO-A2-2007/106356
- WO-A2-2007/133845
- US-A- 5 709 874
- US-A1- 2006 074 308
- US-A1- 2008 281 291

## Description

The present invention relates to a catheter suitable for delivery of a medicament, for example into the bladder.

Catheters for urinary drainage are well-known and include the Foley catheter (or indwelling catheter) and intermittent self-catherization catheter. In each case, the catheter is inserted through the patient's urinary tract into their bladder and allows drainage of urine into a suitable receptacle (eg. container or bag), possibly controlled via a control valve attached to the proximal end of the catheter. A Foley catheter is designed to be left in place in the bladder for a period of time and is held in place by a balloon located at the distal end of the catheter, within the bladder, which is inflated using sterile water following catheter insertion. The inflated balloon is unable to slide down past the bladder neck sphincteric muscles, thus causing retention of the catheter in the urethra. The Foley catheter is therefore a dual-lumen catheter, with one lumen allowing the passage of urine and the second required for inflation of the balloon.

Foley catheters are used in medicine for all bladder drainage situations for both men and women, including during and after surgery. Moreover, Foley catheters may be used to manage urine drainage in non-hospitalised patients where a urine bag or valve to control urine flow can be attached to the proximal end.

However due to its dual-lumen design, the Foley catheter diameter is relatively large, 16 to 18 F being the most common sizes (equating to a diameter of 5.3mm to 5.9mm) and the relatively large size can cause discomfort to the patient. The catheter length is also about 43cm long to accommodate the male urethral length, and is thus inconvenient for use by women.

Major disadvantages with the Foley catheter include the potential for balloon breakage within the patient, failure of the balloon to inflate or, more seriously, premature inflation of the balloon before the catheter is properly inserted into the bladder, which can lead to damage or rupture of the urethra.

In addition to the above difficulties, use of the Foley catheter as a means for delivery of a medicament to the bladder is awkward where only a relatively small volume of medicament is required, due to the relatively large lumen volume.

The intermittent self-catheterization catheter, an alternative to the Foley catheter, is intended for use by patients having an obstructed urethra to catheterize themselves in order to empty their bladder several times a day. These catheters are formed from a straight tube having a rounded distal end and at least one opening near the distal end. Urine drains as soon as the catheter is inserted into the bladder. The tube is generally stiffer than a Foley catheter and is made of a higher durometer material. These catheters cannot be retained in the urethra and typically do not have any valve or other connector at the proximal end which is typically simply the cut end of the tube. Typical examples of these catheters include the 15cm length, 4.6mm (14Fr) diameter catheter (Mentor MR 02403) for women and the 40cm version (Mentor MR 0414) for men.

A known catheter is provided by WO 2007/106356 a which the cathether has a lower with a value to the bladder for introduct of drugs.

The present invention provides a catheter suitable for medicament delivery into a body cavity, such as the bladder.

Aspects of the present invention are defined in claim 1 and the dependent claims.

The catheter of the present invention will now be further described by reference to the following, non-limiting example and figures in which:
Fig 1 is a schematic representation of a catheter according to the present invention which is suitable for use with female patients; and
Fig 2 is a schematic representation of a drainage insert for draining the catheter through the valve.
Fig 3 is a schematic representation of an automatic valve for use with a catheter according to the present invention, displayed firstly (a) in a closed configuration and secondly (b) in an open configuration following insertion of a suitable drainage insert.
Fig 4 is a schematic representation of a catheter according to the present invention which is suitable for use with a male patient.

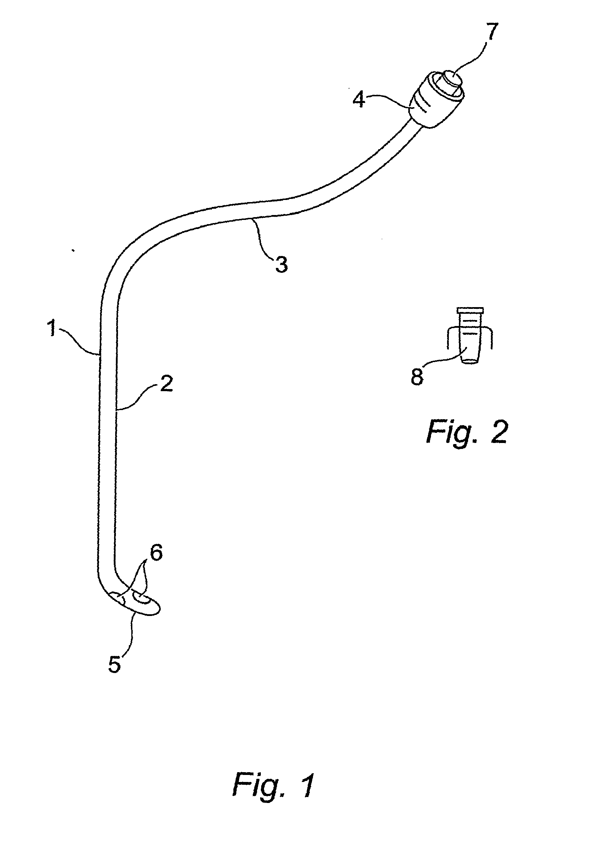

Figure 1 represents a view of a catheter, shown generally at 1, for female patients and having a stiffer distal portion 2 and a softer proximal portion 3. A catheter for male patients will have a longer distal portion 2. A one-way valve 4 is situated at the end of the proximal portion 3 of the catheter 1 and in normal use prevents egress of fluid from the lumen of catheter 1 via aperture 7 at the terminus of proximal portion 3. Distal portion 2 includes an elbow portion 5 to facilitate retention of the catheter within the bladder. Elbow portion 5 includes drainage holes 6 for draining fluids (eg. urine) from the bladder or for medication to be infused into the bladder from the catheter. In use, a syringe (not shown) can be connected to the valve 4 for infusion of medications or for drainage of the bladder. By insertion of a syringe, the valve 4 is forced open to allow fluid passage. Removal of the syringe will allow the valve to automatically close preventing egress of fluids from aperture 7.

Figure 2 represents a simple drainage insert 8. By locating insert 8 into the valve 4, the valve 4 is opened to allow egress of fluid (eg. urine) from the lumen of catheter 2 through aperture 7. A syringe can also be used to open the valve 4.

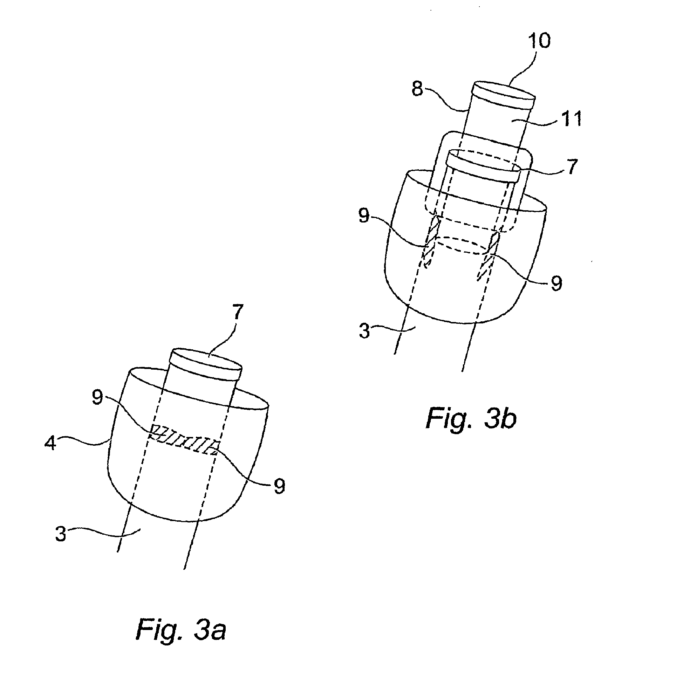

Figure 3 represents a detailed view of a one-way valve 4 located at the proximal end of a catheter (not shown).

Figure 3a) shows the valve 4 in a closed configuration, in which the internal valve flaps 9 (typically of an elastic material) are in an extended configuration, thereby preventing the egress of fluids from the lumen of catheter 3 through aperture 7.

Figure 3b) shows the valve 4 with the drainage insert 8 in place. The drainage insert 8 mechanically urges the valve flaps 9 into a retracted configuration, thereby allowing the egress of fluid from the lumen of catheter 3, through the lumen 11 of drainage insert 8, and out from aperture 10 of drainage insert 8.

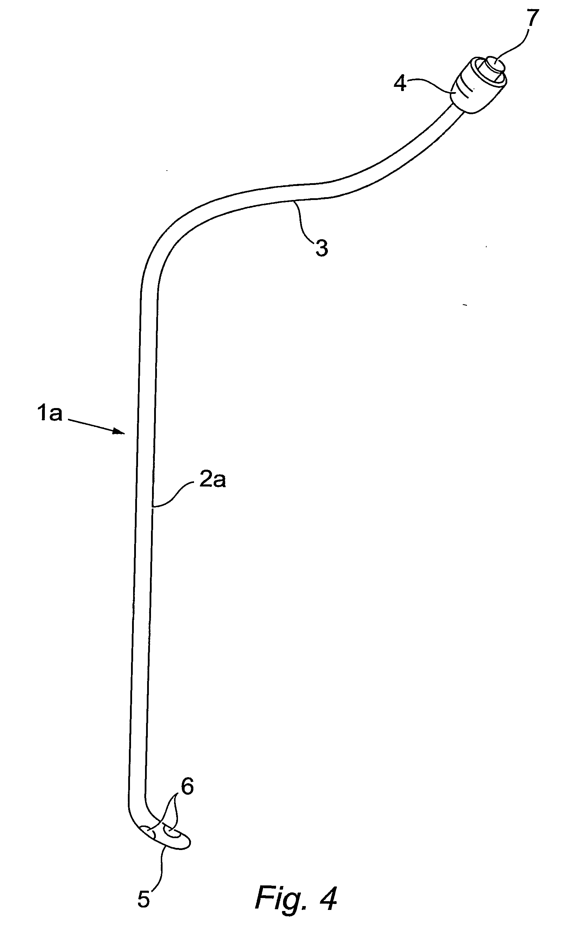

Figure 4 represents a view of a catheter according to the invention, shown generally at 1a, which is suitable for use with male patients. The catheter of Figure 4 differs from the catheter of Figure 1 (which is suitable for use with female patients) only in that stiffer segment 2a is longer than corresponding stiffer segment 2 in the female catheter of Figure 1, and therefore is suitable for use with male patients.

### Example

Preferred Procedure for use: the catheter insertion procedure is to be carried out in an aseptic manner.
(1) The patient is asked to first empty his/her bladder.
(2) Wet the tip of the catheter if it is coated with lubricious hydrogel, or dip the tip into a water soluble jelly such as K-Y jelly.
(3) The catheter is inserted into the bladder
(4) Open the one-way valve by connecting it to a syringe or similar device to drain any remaining urine from the bladder.
(5) Disconnect the drainage syringe/device.
(6) Connect the medication infusion syringe to the valve.
(7) Depress the syringe for medication infusion.
(8) Once the desired infusion in completed, remove the infusion syringe. The removal of the syringe will reactivate the one-way valve automatically preventing any backflow of medication or infusion fluid.
(9) If the medication is to be retained in the bladder for a period of time such as several minutes to several hours, the catheter portion extending outside of the urethral meatus, consisting mostly of the more flexible segment, can be flexed and taped onto the abdomen of the patient.
(10) If needed, repeat infusions of the same solution or other solutions can be done by repeating steps (6) and (7).
(11) If samples of the bladder content are required, withdrawal of the samples can be done as in step (6) except use a syringe for withdrawal of bladder sample rather than infusion.
(12) If the infusion solution is irritative to the urethra, the bladder content can be drained by connecting a syringe barrel or similar device to open the one way valve as in step (4). The bladder content then can be drained without it coming into contact with the urethral wall.
(13) The catheter can then be removed.

## Claims

1. A catheter (1) comprising: i) a tube having a proximal end and a distal end, and a single lumen(11) extending therebetween; ii) at least one opening (6) at said distal end; and there is at least one aperture (7) at said proximal end with a valve (4) at the proximal end arranged on circuit with the lumen(11) **characterised in that** a distal portion (2) of the tube has less flexibility relative to a proximal portion(3) of the tube and the valve is urged in use from a closed configuration by an insert (8) to a retracted configuration

2. The catheter as claimed in claim 1, wherein the valve (4) comprises the valve arranged to prevent egress of fluid from the lumen (11) via the proximal end of the catheter.

3. The catheter as claimed in claim 1 or claim 2, wherein the distal end of the catheter comprises an elbow portion (5).

4. The catheter as claimed in claim 3, wherein the elbow portion (5) is positioned at an angle of 50 [deg.] to 90 [deg.] relative to the catheter tube.

5. The catheter as claimed in any preceding claim, wherein the distal portion of said tube has a durometer of 80 to 90 Shore A, and the proximal portion of said tube has a durometer of 50 to 60 Shore A.

6. The catheter as claimed in any preceding claim, wherein the valve comprises flaps(9) and the flaps are formed from as elastic material.

7. The catheter as claimed in any preceding claim, wherein the valve comprises a male or female luer connector.

8. The catheter as claimed in any preceding claim, wherein said distal end comprises 2 to 4 openings (6).

9. The catheter as claimed in any preceding claim, wherein the catheter has a diameter in the range 3.3 to 4.6mm (10 to 14 F).

10. The catheter as claimed in any preceding claim, wherein the catheter is formed from one or more biocompatible materials selected from the group consisting of latex, silicone, polyethylene, polyolefin, polyvinyl chloride, ethylene vinyl acetate, ethylene propylene diene, polytetrafluoroethylene and a Giomdomptable polyurethane are other biocompatible thermoplastic

11. The catheter as claimed in any preceding claim, wherein the catheter is coated with a lubricant.

12. The catheter as claimed in any preceding claim, further comprising the the

## Patentansprüche

1. Katheter (1), Folgendes umfassend: i) eine Röhre mit einem proximalen Ende und einem distalen Ende und einem einzigen Lumen (11), das sich dazwischen erstreckt, ii) mindestens eine Öffnung (6) am distalen Ende, und es gibt mindestens eine Öffnung (7) am proximalen Ende mit einem Ventil (4) am proximalen Ende, das mit dem Lumen (11) in Verbindung steht, **dadurch gekennzeichnet, dass** ein distaler Abschnitt (2) der Röhre im Verhältnis zu einem proximalen Abschnitt (3) der Röhre eine geringere Flexibilität aufweist und das Ventil im Gebrauch durch einen Einsatz (8) von einer geschlossenen Anordnung in eine zurückgezogene Anordnung gedrückt wird.

2. Katheter nach Anspruch 1, wobei das Ventil (4) das Ventil derart angeordnet umfasst, dass ein Austritt von Fluid aus dem Lumen (11) über das proximale Ende des Katheters verhindert wird.

3. Katheter nach Anspruch 1 oder 2, wobei das distale Ende des Katheters einen gekrümmten Abschnitt (5) umfasst.

4. Katheter nach Anspruch 3, wobei der gekrümmte Abschnitt (5) im Verhältnis zur Katheterröhre in einem Winkel von 50 bis 90 Grad angeordnet ist.

5. Katheter nach einem der vorhergehenden Ansprüche, wobei der distale Abschnitt der Röhre einen Härtegrad von 80 bis 90 Shore A aufweist und der proximale Abschnitt der Röhre einen Härtegrad von 50 bis 60 Shore A aufweist.

6. Katheter nach einem der vorhergehenden Ansprüche, wobei das Ventil Klappen (9) umfasst und die Klappen aus einem elastischen Material gebildet sind.

7. Katheter nach einem der vorhergehenden Ansprüche, wobei das Ventil einen einrückenden oder einen aufnehmenden Luer-Konnektor umfasst.

8. Katheter nach einem der vorhergehenden Ansprüche, wobei das distale Ende 2 bis 4 Öffnungen (6) umfasst.

9. Katheter nach einem der vorhergehenden Ansprüche, wobei der Katheter einen Durchmesser im Bereich von 3,3 bis 4,6 mm (10 bis 14 F) aufweist.

10. Katheter nach einem der vorhergehenden Ansprüche, wobei der Katheter aus einem oder mehreren biokompatiblen Materialien gebildet ist, die aus der Gruppe ausgewählt sind, die besteht aus: Latex, Silikon, Polyethylen, Polyolefin, Polyvinylchlorid, Ethylenvinylacetat, Ethylenpropylendien, Polytetrafluorethylen und einem biokompatiblen Polyurethan oder einem anderen biokompatiblen thermoplastischen Kunststoff.

11. Katheter nach einem der vorhergehenden Ansprüche, wobei der Katheter mit einem Schmiermittel beschichtet ist.

12. Katheter nach einem der vorhergehenden Ansprüche, ferner den Einsatz (8) umfassend.

## Revendications

1. Sonde (1) comportant : i) un tube présentant une extrémité proximale et une extrémité distale, et une seule lumière (11) s'étendant entre les deux ; ii) au moins une ouverture (6) au niveau de ladite extrémité distale ; et au moins une ouverture (7) existante au niveau de ladite extrémité proximale avec une valve (4) au niveau de l'extrémité proximale agencée dans le circuit avec la lumière (11) **caractérisée en ce qu'**une partie (2) distale du tube a une moins grande flexibilité par rapport à une partie (3) proximale du tube et la valve est amenée, lors de l'utilisation, à passer d'une configuration fermée par un insert (8) à une configuration rétractée.

2. Sonde selon la revendication 1, dans laquelle la valve (4) comprend la valve agencée de manière à empêcher les fluides de sortir de la lumière (11) par l'extrémité proximale de la sonde.

3. Sonde selon la revendication 1 ou la revendication 2, dans laquelle l'extrémité distale de la sonde comporte une partie (5) coudée.

4. Sonde selon la revendication 3, dans laquelle la partie (5) coudée est placée à un angle de 50 [degrés] à 90 [degrés] par rapport au tube de sonde.

5. Sonde selon l'une quelconque des revendications précédentes, dans laquelle la partie distale dudit tube a une dureté de l'ordre de 80 à 90 à l'échelle Shore A, et la partie proximale dudit tube a une dureté de l'ordre de 50 à 60 à l'échelle Shore A.

6. Sonde selon l'une quelconque des revendications précédentes, dans laquelle la valve comporte des clapets (9) et les clapets sont constitués d'un matériau élastique.

7. Sonde selon l'une quelconque des revendications précédentes, dans laquelle la valve comporte un raccord Luer mâle ou femelle.

8. Sonde selon l'une quelconque des revendications précédentes, dans laquelle ladite extrémité distale comporte de 2 à 4 ouvertures (6).

9. Sonde selon l'une quelconque des revendications précédentes, dans laquelle la sonde a un diamètre compris dans la plage allant de 3,3 à 4,6 mm (10 à 14 F).

10. Sonde selon l'une quelconque des revendications précédentes, dans laquelle la sonde est constituée d'un ou plusieurs matériaux biocompatibles choisis dans le groupe comprenant du latex, de la silicone, du polyéthylène, de la polyoléfine, du chlorure de polyvinyle, de l'acétate d'éthylène-vinyle, de l'éthylène-propylène-diène, du polytétrafluroéthylène et du polyuréthane biocompatible ou autre thermoplastique biocompatible.

11. Sonde selon l'une quelconque des revendications précédentes, dans laquelle la sonde est revêtue de lubrifiant.

12. Sonde selon l'une quelconque des revendications précédentes, comportant en outre un insert (8).
